# EUROPÄISCHE PATENTSCHRIFT

(11) **EP 1 652 431 B1**
(45) Veröffentlichungstag und Bekanntmachung des Hinweises auf die Patenterteilung: **06.11.2013**
(21) Anmeldenummer: 05019735.9
(22) Anmeldetag: 10.09.2005
(51) Int. Cl.: A01N 37/12, A01N 37/02, A23D 9/06, A23L 1/24, C11D 3/48, A61K 8/39, A61Q 11/00, A61Q 15/00, A61Q 17/00, A61Q 19/10, A23L 3/3508, A23L 3/3517, A23L 3/3562

(54) **Mittel mit Depotwirkung zur Bekämpfung von Mikroorganismen**
Agents having a controlled sustained release to combat microorganisms
Agents à effet dépôt pour lutter contre les micro-organismes

(30) Priorität: 25.09.2004 DE 102004046603
(43) Veröffentlichungstag der Anmeldung: 03.05.2006
(73) Patentinhaber: Evonik Degussa GmbH, 45128 Essen (DE)
(72) Erfinder: Allef, Petra, Dr., 45147 Essen (DE); Bergfried, Stefan, 45134 Essen (DE); Grüning, Burghard, Dr., 45134 Essen (DE); Weitemeyer, Christian, Dr., 45257 Essen (DE)

(56) Entgegenhaltungen:
- EP-A1- 1 250 842
- EP-A2- 0 465 423
- WO-A1-01/97799
- WO-A1-94/09753
- WO-A1-95/07616
- WO-A1-2005/023023
- WO-A2-01/43549
- DE-A1- 2 755 052
- US-A- 5 569 461
- DATABASE WPI Week 197906 Thomson Scientific, London, GB; AN 1979-10871B XP002666228, & JP 53 148528 A (TAKEDA CHEM IND LTD) 25. Dezember 1978 (1978-12-25)
- DATABASE WPI Week 199442 Thomson Scientific, London, GB; AN 1994-337389 XP002666229, & JP 6 261725 A (TAIYO KAGAKU KK) 20. September 1994 (1994-09-20) -& DATABASE CA [Online] CHEMICAL ABSTRACTS SERVICE, COLUMBUS, OHIO, US; XP002666230, gefunden im STN-International accession no. 122:79687 & JP 6 261725 A (TAIYO KAGAKU KK) 20. September 1994 (1994-09-20)
- DATABASE WPI Week 199619 Thomson Scientific, London, GB; AN 1996-182268 XP002666231, & JP 8 056631 A (DAIICHI KOGYO SEIYAKU CO LTD) 5. März 1996 (1996-03-05)

## Beschreibung

Gegenstand der Erfindung sind Mittel mit Depotwirkung zur Bekämpfung von Mikroorganismen, enthaltend einen wirksamen Gehalt an Fettsäureestern des Polyglycerins und an Salzen von kurzkettigen Monocarbonsäuren ausgewählt aus Caprylsäure.

Zur Bekämpfung von Mikroorganismen (grampositive Bakterien, gramnegative Bakterien, Mykobakterien, Dermatophyten, Spross-und Fadenpilze, Viren und Sporen) welche an der Oberfläche von Haut und Haaren, Kleidung, Geräten der Körperreinigung und -pflege wie beispielsweise im Dentalbereich, medizinischen Instrumenten aber auch Räumen und Einrichtungsgegenständen vorhanden sind, sind eine Vielzahl antimikrobiell wirksamer chemischer Substanzen bzw. Gemische dieser Substanzen bekannt, welche nach ihrem Verwendungszweck in Desinfektionsmittel, Konservierungsmittel, Antiseptika und kosmetische Wirkstoffe, um einige aufzuzählen, eingeteilt werden.

Die wesentlichen Vertreter dieser Gruppen sind: Aldehyde wie Formaldehyd, Glyoxal oder Glutaraldehyd; Phenol-Derivate wie 2.2`-Dihydroxybiphenyl und 4-Chlor-3-methylphenol; quaternäre Ammoniumverbindungen, Kationentenside wie Benzalkoniumchlorid, Cetrimoniumbromid, Cetylpyridiniumchlorid; Amphotenside sowie aktiven Sauerstoff abspaltende Verbindungen wie beispielsweise Wasserstoffperoxid, organische Persäuren, Alkylperoxide oder Alkylhydroperoxide.

Diese weisen allerdings etliche Nachteile auf, da sie die vielfältigen Forderungen, die in der Praxis an sie gestellt werden, wie z. B. breites Wirkungsspektrum, kurze Einwirkungszeiten bei niedrigen Temperaturen, gute Hautverträglichkeit, geringe Toxizität, Materialverträglichkeit, nicht oder nur unzureichend erfüllen.

Desinfektionsmittel auf Aldehyd- oder Phenolbasis gelten als toxikologisch und ökologisch bedenklich, führen häufig zu Sensibilisierungen insbesondere der Haut und Atmungsorgane und weisen darüber hinaus einen charakteristischen, durchdringenden und unangenehmen Geruch auf. Einige gelten zudem als potentielle Kanzerogene.

Quaternäre Ammoniumverbindungen (Quats) sind toxikologisch weitestgehend unbedenklich, weisen keine oder nur sehr geringere Hautsensibilisierung auf und sind praktisch geruchlos. Sie besitzen jedoch eine beträchtliche hautreizende Wirkung. Bei Einsatz von Quats können sich wie bei der Verwendung von Aldehyden unerwünschte Ablagerungen und Schichten auf den behandelten Flächen bilden, die optisch nachteilig in Erscheinung treten und sich durch übliche Reinigungsverfahren nur schwer oder überhaupt nicht wieder entfernen lassen.

Aus der DE-A-42 37 081 sind kosmetische Deodorantien bekannt, welche als Wirkstoffe Fettsäureester des Di- und Triglycerins enthalten. Nach der dort gegebenen Lehre sind nur die Monoester wirksam zur Bekämpfung grampositiver Bakterien.

Diese Monoester können gemäß den bekannten chemischen Verfahren des Standes der Technik (DE-A-38 18 293) hergestellt werden durch alkalisch katalysierte Umsetzung eines 1,5 bis 2,5-fachen molaren Überschusses an Fettsäuren oder Fettsäurederivaten mit Isopropylidenderivaten des Di- und Triglycerins, nachfolgender Reinigung des Reaktionsproduktes und anschließender saurer Hydrolyse oder Alkoholyse der Isopropylidengruppen.

Daneben sind auch enzymatisch katalysierte Verfahren zur Herstellung von Polyglycerinfettsäureestern bekannt. D. Charlemagne und M. D. Legoy (JAOCS 1995, Vol. 72, no. 1, 61-65) adsorbieren hierzu erst das Polyglycerin auf der gleichen Menge Kieselgel, bevor sie in Suspension mit Fettsäuremethylestern unter Lipasekatalyse reagieren lassen. Nachteilig ist hierbei vor allem der Verlust des teuren Enzyms, das nach Beendigung der Reaktion zusammen mit dem Kieselgel durch Filtration abgetrennt wird. S. Matsumura, M. Maki, K. Toshima und K. Kawada (J. Jpn. Oil Chem. Soc. 1999, Vol. 48, No. 7, 681-692) nutzen dieses Verfahren in Abwandlung, um unter Einsatz von 20 Gew.-% Enzym Polyglycerinester zu synthetisieren. Gemäß der in der DE-A-42 37 081 vermittelten Lehre reinigen sie unter hohem Aufwand mittels Säulenchromatographie weiter auf, um zu reinen Monoestern mit den bekannten antimikrobiellen Aktivitäten zu gelangen.

Nach EP-B-1 250 842 sind Mischungen von Fettsäuremono-, -di- und -triestern des Polyglycerins, welche durch enzymatisch katalysierte Umsetzung hergestellt wurden, bekannt. Diese sollen vergleichbare und teilweise sogar deutlich bessere Wirksamkeiten bei der Bekämpfung von Mikroorganismen aufweisen, als die durch chemische Synthese oder enzymatische Herstellung und Aufreinigung hergestellten Monoester.

Die EP0465423 offenbart pharmazeutische Zusammensetzungen enthaltend C4 bis C14 Fettsäuren und ihre Monoglyceride.

Die WO 01/97799 offenbart die Verwendung von C6 bis C10 Fettsäuren, deren Salze, Derivate oder Mischungen davon zur Inhibierung des Wachstums mikorbieller Kontaminationen, Wachstum und/oder daran angeschlossener Toxin-Produktion.

Die JP6261725 offenbart Konservierungsmittel für Nahrungsmittel henthaltend Ploylgylcerinfettsäureester sowie organische Säuren und/oder deren Salze.

Aufgabe der Erfindung war es daher, Mittel zur Bekämpfung von Mikroorganismen zu finden, die den geschilderten Nachteilen der Mittel des Standes der Technik weitgehend Abhilfe schaffen, eine hohe antimikrobielle Wirkung zeigen und aus einfach zugänglichen Rohstoffen nach einem ökonomisch tragbaren und ökologisch unbedenklichen Verfahren unkompliziert hergestellt werden können.

Überraschenderweise wurde gefunden, dass die antimikrobielle Wirkung der aus dem Stand der Technik bekannten Polyolester von Mischungen aus kurzkettigen Fettsäuren bzw. deren Salzen und den Polyolestern deutlich übertroffen wird und somit verbesserte antimikrobielle Mittel bereitgestellt werden können. Ohne die Erfindung auf einen Mechanismus beschränken zu wollen, so kann man doch die Wirkung der Polyolester wesentlich auf die Spaltung der Ester durch auf der Haut vorkommenden Enzyme zurückführen. Die Tatsache, dass die Spaltung der Ester erst in hinreichendem Maße eintritt, wenn genügend Mikroorganismen vorhanden sind, führt dazu, dass die Wirkung der mit Polyolestern hergestellten Deoformulierungen oftmals zu spät einsetzt, um die Bildung störender Köpergerüche komplett zu verhindern. Aus diesem Grunde waren weitere antimikrobielle Wirkstoffe als Zusatz nötig.

Ein Gegenstand der Erfindung sind daher antimikrobiell wirksame Mittel zur Bekämpfung von Mikroorganismen, welche gekennzeichnet sind durch einen wirksamen Gehalt an Mischungen von Fettsäureestern, insbesondere Fettsäuremono- und -diestern des Polyglycerins, insbesondere des Mono-, Di- und/oder Triglycerins mit C₆₋₁₄ Monocarbonsäuren und von Salzen kurzkettiger, Monocarbonsäuren, ausgewählt aus Caprylsäure.

Der Einsatz von kurzkettigen Fettsäuren allein ist wenig wünschenswert, da diese unangenehm riechen und in höheren Dosen hautreizend sind. Des Weiteren werden sie schnell auf der Haut metabolisiert und bieten damit keinen Langzeitschutz. (Kabara JJ. Fatty acids and derivatives as antimicrobial agents. In: Kabara JJ, ed. The Pharmacological Effect of Lipids I. Champaign, IL: American Oil Chemists' Society; 1978; 1-14. Wyss O, Ludwig BJ, Joiner RR. The fungistatic and fungicidal action of fatty acids and related compounds. Arch Biochem. 1943;7,415.)

Ein weiterer Gegenstand der Erfindung ist die Verwendung dieser antimikrobiell wirksamen Mischungen zur Herstellung von Desinfektionsmitteln, Sterilisationsmitteln, Antiseptika, Konservierungsmitteln, die sich zur Sterilisation und Desinfektion von Flächen und chirurgischen Instrumenten sowie zur Konservierung, insbesondere zur Konservierung von kosmetischen oder dermatologischen Zubereitungen, eignen.

Die Mittel eignen sich darüber hinaus auch zur Konservierung von Lebensmitteln und können auch zur antimikrobiellen Ausrüstung von Lebensmittelverpackungen eingesetzt werden. Die erfindungsgemäßen antimikrobiellen Mittel eignen sich insbesondere, auch aufgrund ihrer Mildheit, zur Herstellung kosmetischer Präparate zur Bekämpfung des Körpergeruches, zur Bekämpfung von Schuppen und zur Bekämpfung unreiner Haut und zur Bekämpfung von Karies.

Weitere Gegenstände der Erfindung sind durch die Ansprüche gekennzeichnet.

Bevorzugt sind Polyglycerine mit der allgemeinen Formel

HO-CH₂-CH(OH)-CH₂-O-[CH₂-CH(OH)-CH₂-O]ₙ-H

worin n = 0 bis 9, vorzugsweise 1 bis 6, insbesondere 1 bis 3, speziell 1 und 2 bedeutet. Darüber hinaus können die verwendeten Polyglycerine auch verzweigt sein und cyclische Anteile enthalten.

Es sind bei Raumtemperatur hochviskose Flüssigkeiten, welche neben Diglycerin vor allem die höher kondensierten Oligomeren des Glycerins enthalten. Besonders bevorzugt im Sinne der vorliegenden Erfindung werden technische Gemische von Polyglycerinen eingesetzt, die üblicherweise Diglycerin, Triglycerin, Tetraglycerin und Pentaglycerin enthalten.

Sie können beispielsweise industriell hergestellt werden durch basenkatalytische Kondensation von Glycerin oder auch durch Hydrolyse und Kondensation von Epichlorhydrin. Darüber hinaus sind Polyglycerine auch zugänglich durch Polymerisation von Glycidol. Die Trennung und Isolation der einzelnen Polyglycerine ist durch Behandlung mit den verschiedenen im Stand der Technik, bekannten Mitteln möglich. Eine Übersicht von G. Jakobson über die verschiedenen Syntheserouten ist zu finden in "Fette Seifen Anstrichmittel", 1986, 88. Jahrgang, Nr. 3, 101-106. Die verschiedenen Strukturmöglichkeiten für Polyglycerin können nachgelesen werden bei H. Dolhaine, W. Preuß und K. Wollmann (Fette Seifen Anstrichmittel 1984, 86. Jahrgang, Nr. 9, 339-343).

Handelsübliche Produkte sind im Allgemeinen Gemische von Polyglycerinen mit verschiedenen Kondensationsgraden, der maximale Kondensationsgrad kann darin in der Regel bis zu 10 in Ausnahmen auch größer sein. Besonders bevorzugt werden Polyglycerine, die nur oder überwiegend Di- und Triglycerin enthalten, verwendet.

Die im Sinne der vorliegenden Erfindung vorzugsweise einzusetzenden Fettsäuren und Fettsäurederivate sowie deren Gemische für die Esterbildung leiten sich von gradkettigen oder verzweigten, gesättigten, einfach oder mehrfach ungesättigten Carbon- und Fettsäureresten mit 6 bis 14 C-Atomen, vorzugsweise 8 bis 12, insbesondere 8 bis 10 C-Atomen in der Hauptkette ab.

Als Fettsäurederivate können alle üblichen Derivate eingesetzt werden, die Ver-/Umesterungsreaktionen eingehen. Erfindungsgemäß sind die Fettsäurederivate besonders bevorzugt ausgewählt aus Fettsäurealkylestern mit 1 bis 4 C-Atomen im Alkoholrest.

Als Fettsäuren oder deren Ester werden einzeln oder in Mischungen Fettsäuren wie Capronsäure, Caprylsäure, Caprinsäure, 2-Ethylhexansäure, Undecylensäure, Laurinsäure und Myristinsäure verwendet. Geeignet sind prinzipiell alle Fettsäuren mit ähnlicher Kettenverteilung.

Vorzugsweise werden Caprylsäure und Caprinsäure verwendet.

Die Ester werden hergestellt nach einem im Stand der Technik bekannten chemischen oder enzymatischen Verfahren, wie beispielsweise in der DE-B-42 37 081, EP-B-1 250 842 oder EP-B-0 451 461.

Die erfindungsgemäßen Polyolfettsäureester bestehen zusammenfassend aus einem Gemisch von Verbindungen unterschiedlichen Veresterungsgrades, das beträchtliche Anteile nicht veresterten Polyols enthalten kann. Das zu Grunde liegende Polyol kann dabei einheitlich oder seinerseits wiederum ein Gemisch von Produkten unterschiedlichen Kondensationsgrades sein.

Ein weiterer wesentlicher Bestandteil der erfindungsgemäßen Mischungen sind Salze von Diese Salze sind bei den physiologischen Gegebenheiten der Haut sofort und so lange ausreichend wirksam bis die deodorierende Wirkung aus dem Depot der Estern eintritt.

Diese Salze können Alkali-, Erdalkali- und/oder Ammoniumsalze der Säuren sein. Zur Herstellung klarer Lösungen der Salze in den Estern werden erfindungsgemäß bevorzugt die Alklisalze, insbesondere die Kaliumsalze mitverwendet.

Das Mischungsverhältnis von Ester und Salz ist im Grunde unkritisch und kann in weiten Teilen variiert werden. Da jedoch eine maximale Depotwirkung mit hinreichender Sofortwirkung angestrebt wird, ist im Algemeinen ein Anteil von 1 bis 20 % insbesondere 5 bis 10 % der Salze ausreichend.

Die erfindungsgemäßen Mittel zur Bekämpfung von Mikroorganismen können je nach Einsatzzweck darüber hinaus noch auf diesem Gebiet übliche anionische, nichtionische, kationische und/oder amphotere Tenside enthalten.

Typische Beispiele für solche Tenside sind:
1. Nichtionische Tenside basierend auf Alkylenoxiden wie Ethoxylate langkettiger verzweigter Alkohole, Ethoxylate von Sorbitanestern, Propylenoxidethylenoxidcopolymere, Hydroxyalkylfettsäureamide, Polydimethylsiloxanpolyalkylenoxidcopolymere, Zucker basierte Tenside wie Alkylpolyglycoside, Alkylglycosidester, N-Acylglucamide und Polyglycerinester,
2. Anionische Tenside wie Alkyl- und Alkylethersulfate, α-Olefinsulfonate, Fettsäureestersulfonate, Alkylarylsulfonate, Sulfosuccinate, Phosphorsäurealkyl- oder alkoxyalkylester, Taurate, N-Acylaminosäurederivate, Sarcosinate, Isethionate und Seifen,
3. Kationische Tenside wie Alkyltrimethylammoniumsalze, Fettsäureester von Di- oder Triethanolammoniumsalzen, Alkylimidazoliniumsalzen, Acylamidopropyldimethylammoniumsalze, kationisch derivatisierte Polydimethylsiloxane,
4. Zwitterionische und amphotere Tenside wie Betaine, Sulfobetaine, Aminoxide und Amphoacetate.

Bei den erfindungsgemäßen Mitteln zur Bekämpfung von Mikroorganismen handelt es sich beispielsweise um Sterilisationsmittel, Desinfektionsmittel, desinfizierende Reinigungsmittel, Allzweckreiniger, Sanitärreiniger, Badreiniger, Maschinengeschirrspülmittel, Waschmittel, kosmetische Reinigungs- und Pflegemittel. Kosmetische Mittel auf Basis der beschriebenen Polyolfettsäureester werden insbesondere in Mengen von 0, 01 bis 5 .Gew.'-% eingesetzt zur Bekämpfung von Körpergeruch, von Schuppen, von Unreinheiten der Haut oder Karies. Sie können als solche in Form homogener Flüssigkeiten, als Gele, als Salben, als Pasten, als wachsartige oder emulsionsartige Zubereitungen formuliert sein. Auch die Verwendung in Form feuchter Tücher ist möglich. Insbesondere in der Emulsionsform werden sie Öle wie Esteröle, flüchtige oder weniger flüchtige Siliconderivate wie Decamethylcyclopentasiloxan, Paraffinöle und dergleichen enthalten.

Auf die zusätzliche Mitverwendung von im Stand der Technik üblicherweise zur Bekämpfung von Mikroorganismen eingesetzten, notwendigen antimikrobiell wirksamen Stoffen kann bei Anwedungen der erfindungsgemäßen Mischungen, insbesondere im pH-Bereich < 7, verzichtet werden. Dies ist aber, falls erwünscht, im Wesentlichen ohne Nachteile möglich.

Als solche seien genannt Triclosan, Farnesol, 2-Ethylhexyloxyglycerin oder Octyllactat. Sie können je nach Anwendungszweck neben den genannten Tensiden noch die jeweils spezifischen Hilfs- und Zusatzstoffe beispielsweise Lösungsmittel, Gerüststoffe, Schauminhibitoren, Salze, Bleichmittel, Bleichaktivatoren, optische Aufheller, Vergrauungsinhibitoren, Lösungsvermittler, Verdickungsmittel, Duft- und Farbstoffe, Emulgatoren, biogene Wirkstoffe wie Pflanzenextrakte und Vitaminkomplexe enthalten. Als Lösungsmittel kommen insbesondere in Frage Wasser oder Alkohole wie beispielsweise Ethanol, Propanol, Isopropanol, 2-Methyl-2-propanol, Propylenglykol, Dipropylenglykol oder Glycerin.

Die jeweils einzusetzenden Mengen an derartigen Zusatzstoffen sind in Abhängigkeit von der Art des jeweiligen Produktes dem Fachmann bekannt oder können im Bedarfsfall durch einfaches Ausprobieren leicht ermittelt werden.

Weitere Verwendungsmöglichkeiten für die erfindungsgemäßen Mittel ist deren Einsatz als Konservierungsmittel in Lebensmitteln und in Lebensmittelverpackungen, in denen sie in der Regel in Konzentrationen von 0,01 bis 5 Gew.-%, bevorzugt 0,1 bis 1 Gew.-% eingesetzt werden. Lebensmitteln können die erfindungsgemäßen Ester einfach in entsprechender Menge zugesetzt werden. Zur Anwendung in Verpackungen kommen die Polyolester, indem beispielsweise Papiere mit einer Lösung oder Emulsion der Ester getränkt werden oder Folien mit entsprechenden Zubereitungen der Ester besprüht werden. Die Ester können auch vor oder während des Formgebungsprozesses der Verpackungen wie der Extrusion zugesetzt werden.

Die erfingungsgemäßen Mischungen werden vorzugsweise hergestellt indem man das Polyol mit Fettsäure, vorzugsweise im Molverhältnis 1 : 1 ohne Lösungsmittel unter Inertatmosphäre bei Temperaturen von 180 bis 260 °C nach an sich bekannten Verfahren verestert. Bei einem Umsetzungsgrad von 90 bis 95 % der Fettsäure wird auf Temperaturen von vorzugsweise < 100 °C abgekühlt und mit einer Base, vorzugsweise den Carbonaten, insbesondere mit Kaliumcarbonat neutralisiert.

Ebenso ist die vollständige Veresterung in 1. Stufe und die nachträgliche Zugabe der Säuren und deren Neutralisation in 2. Stufe bzw. die Zugabe der Salze der Säuren möglich.

Die nachfolgenden Ausführungsbeispiele stellen bevorzugte Umsetzungen der vorliegenden Erfindung dar, sind jedoch nicht geeignet, die Erfindung hierauf zu beschränken.

### Mikrobiologische Tests:

Die Wirksamkeit der erfindungsgemäßen Produkte wird mit Hilfe der "Prüfung auf ausreichende Konservierung" (gemäß der Europäischen Pharmaverordnung) festgestellt. Hierbei zeigt sich, dass die erfindungsgemäßen Produkte gegenüber dem Stand der Technik weit überlegen sind.

### Durchführung der mikrobiologischen Tests:

### A) Gegen Corynebacterium xerosis, Staphylococcus epidermidis und Candida albicans

### 1. Proben und Material:

### 1.1. Proben

a. Diglycerincaprylat (Vergleichssubstanz gemäß dem Stand der Technik)
b. Diglycerincaprylat mit 5 % Kaliumcaprylat
c. Diglycerincaprylat mit 10 % Kaliumcaprylat
d. Diglycerincaprylat mit 15 % Kaliumcaprylat
e. Polyglycerin-3 caprylat Vergleichssubstanz
f. Polyglycerin-3 caprylat mit 7 % Kaliumcaprylat
g. Triethyleneglykol-caprylat Vergleichssubstanz
h. Triethyleneglykol-caprylat mit 5 % Kaliumcaprylat Vergleichsmischung
i. Sorbitancaprylat Vergleichssubstanz
j. Sorbitancaprylat mit 5 % Kaliumcaprylat Vergleichsmischung

### 1.2.Testkeime

Corynebacterium xerosis DSM 20743
Staphylococcus epidermidis DSM 3269
Candida albicans ATCC 10231

### 1.3.Verwendete Medien

Nährmedien:
CSL: Caseinpepton-Sojamehlpepton-Lösung
CSA: Caseinpepton-Sojamehlpepton-Agar
Sabouraud-Glucose-Bouillon/Agar
Verdünnungsflüssigkeit mit Inaktivierungszusätzen
NaCl-Pepton-Pufferlösung mit EG-Enthemmer (3 % Tween® 80, 0,3 % Lecithin, 0,1 % Histidin, 0,5 % Na-Thiosulfat)

### 2. Methode

### 2.1. Herstellen der Testlösungen

Von jeder Probe wurden am Vortag der Untersuchung Testlösungen von 0,1% (w/v) in CSL angesetzt. Dazu wurden je 100 ml CSL im Wasserbad auf 60 °C temperiert. Von jeder Probe wurde 0,1 g in jeweils 100 ml CSL von 60 °C eingewogen. Die Ansätze wurden von Hand kräftig geschüttelt und bei 30 °C über Nacht im Brutschrank belassen.

### 2.2.Herstellen der Testkeimsuspensionen

Corynebacterium xerosis über 3 bis 4 Tage anzüchten. Übrige Keime in Bouillon bzw. durch Abschwemmen gewinnen.

### 2.3. Kontamination der Proben und Ermittlung der Keimzahlreduktion

Von jeder Testlösung wurden für jeden Testkeim 20 ml in sterile 50 ml Braunglasflaschen mit Glasperlen abgefüllt und mit 0,2 ml Keimsuspension kontaminiert. Als Kontrollen wurden je Testkeim 20 ml CSL ohne Probe mitgeführt. Die kontaminierten Proben wurden auf der Schüttelmaschine 3 min geschüttelt und bis zu den Entnahmen bei 30 °C im Brutschrank gehalten.

Zu den Entnahmezeitpunkten (1, 2, 3, 24 und 48 Stunden) wurde aus jedem Ansatz 1 ml entnommen, in jeweils 9 ml NaCl-Pepton-Pufferlösung mit EG-Enthemmer überführt und die Koloniezahl ermittelt.

Als 0-Stunden-Werte wurden die Koloniezahlen der verwendeten Testkeimsuspension unter Berücksichtigung der 10⁻²-Verdünnung bei der Probenkontamination angegeben.

### 3. Ergebnisse

Die Einzelergebnisse der Proben sind in folgenden Diagrammen dargestellt. Zusätzlich sind in jedem Diagramm die Keimpopulationen einer wirkstofffreien Blindprobe als (isolierten) Kontrollwert nach 24 Stunden Bebrütung eingetragen.

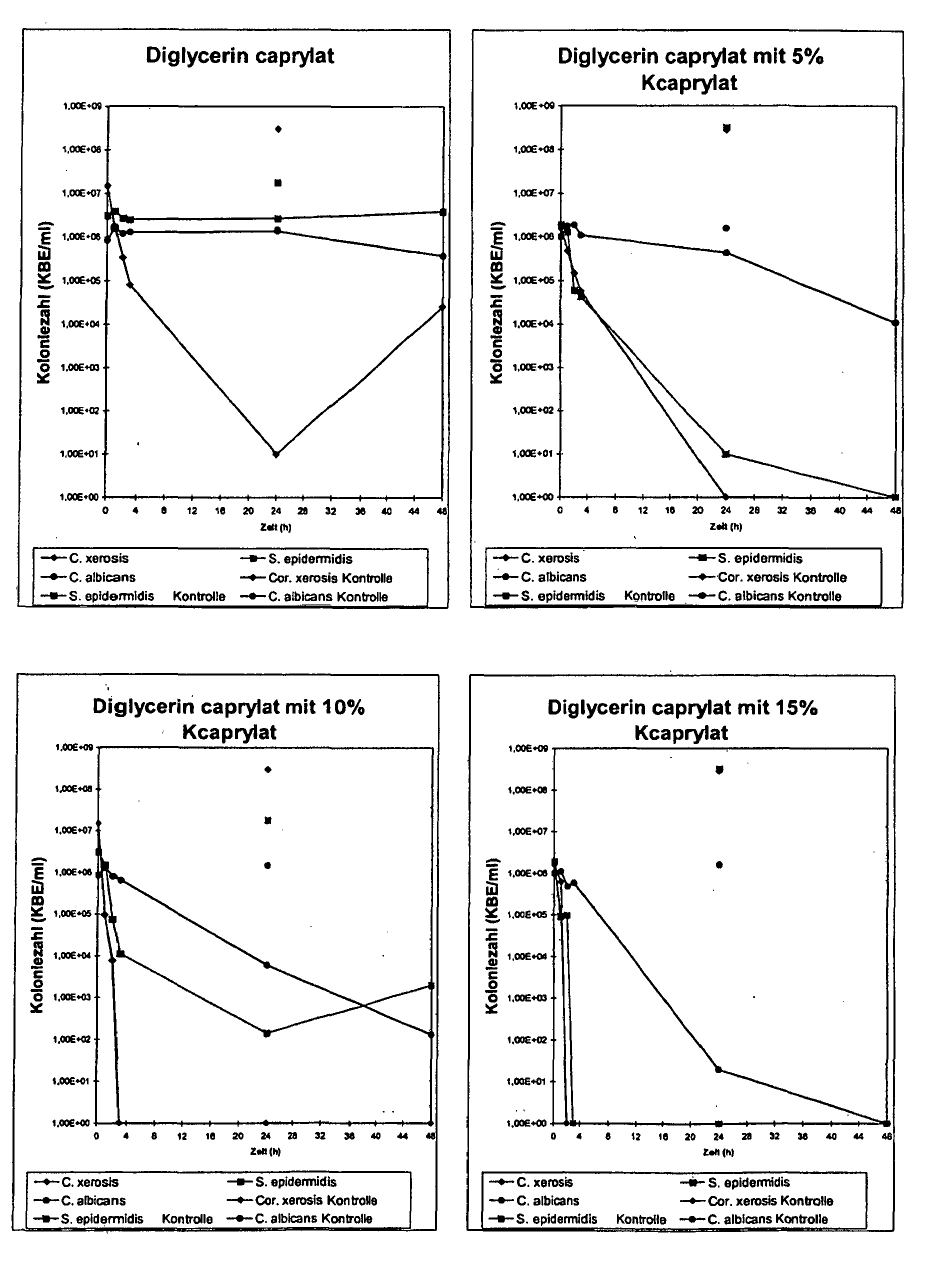

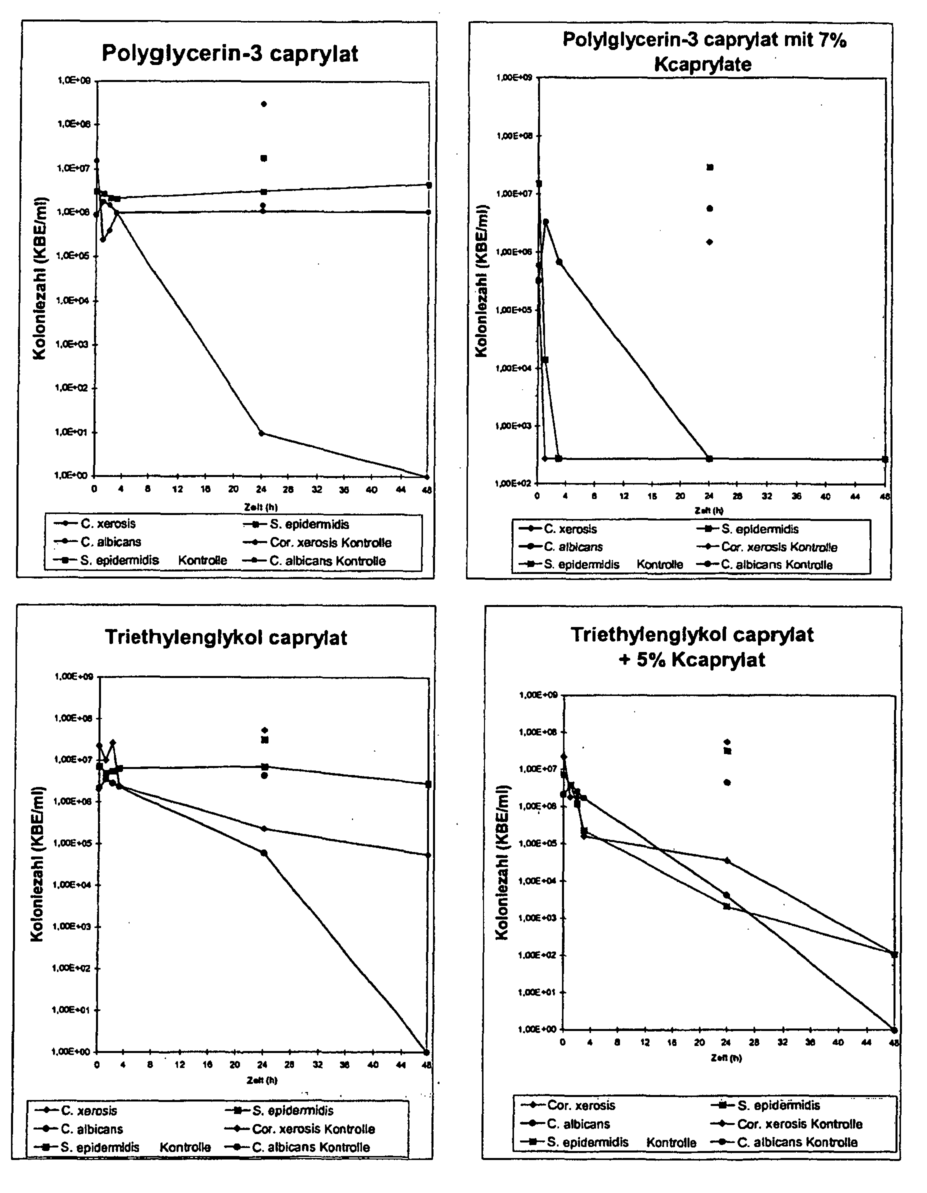

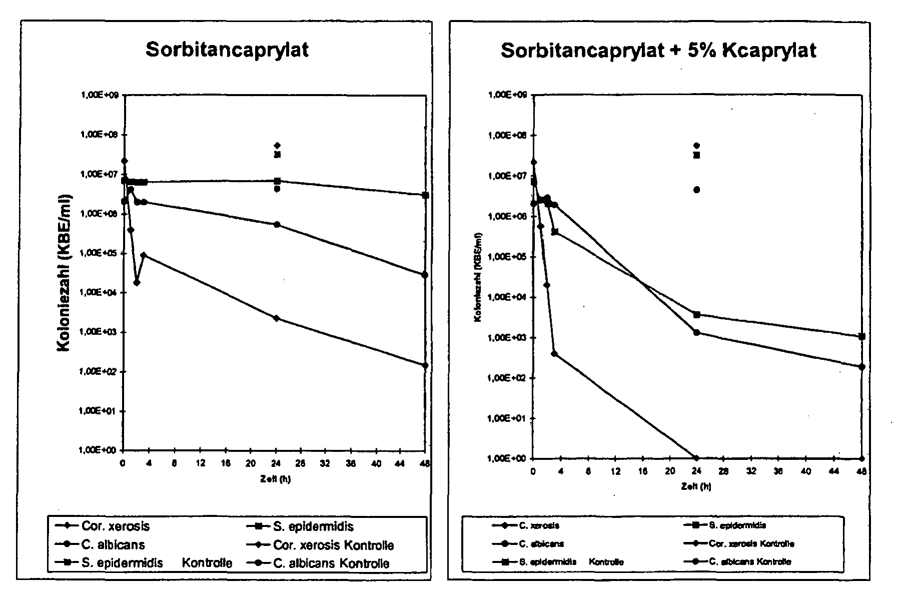

### B) Gegen Malassezia furfur

In gleicher Vorgehensweise wie unter A beschrieben wird die Wirksamkeit von Diglycerincaprylat, enthalten 7 % Kaliumcaprylat, gegen M. furfur getestet. M. furfur steht in ursächlichem Zusammenhang mit der Bildung von Schuppen.

Das erfindungsgemäße Gemisch wurde in Wasser gelöst, so dass eine Lösung mit 3,0 Gew.-% Gehalt entsteht. Diese Lösung wird mit Keimsuspension versetzt, durch Schütteln homogenisiert und bei 30 °C im Brutschrank gehalten. Eine zweite Lösung ohne Zusatz von Diglycerincaprylat wird als Kontrolle mitgeführt.

### Die folgenden Resultate wurden erhalten:

| Probenahme, Zeit (h) | 0 | 1 | 2 | 4 | 24 |
|---|---|---|---|---|---|
| Kontrolle, Keimzahl/ml | 1 x 10⁵ | n.b. | n.b. | n.b. | 1 x 10⁴ |
| 0,3 % Diglycerincaprylat, Keimzahl/ml | 1 x 10⁵ | < 10 | < 10 | < 10 | < 10 |

| | | | | | |
|---|---|---|---|---|---|
| n.b. = nicht bestimmt | | | | | |

### Nachweis der Spaltbarkeit von Polyolestern durch Hautmikroben:

Mikroorganimsmen wurden mittels eines Achselabstriches gesammelt. Dazu wurde mit einem in Pufferlösung (Acetat-Puffer (0,1 M, pH 5,6), enthaltend 0,1 Gew.-% Triton X100) getränktem Wattestäbchen die Haut in der Achselhöhle ca. eine Minute lang abgerieben. Das Wattestäbchen wurde dann in eine Lösung von 2-hydroxy-4-p-nitrophenoxy-butyl-decanoat gegeben. Dieser Ester wurde in 1,5 h durch die von den Hautmikroben exprimierten Enzymen gespalten. Die Spaltmoleküle lassen sich leicht durch Oxidation mit NaIO₄ und Spaltung mit BSA in p-Nitrophenol überführen, das mittels UV-Spektroskopie quantifiziert werden kann. Vergleiche D. Lagarde, H.K. Nguyen, G. Ravot, D. Wahler, J.-L. Reymond, G. Hills, T. Veit, F. Lefevre, Org. Process Res. Dev., 6, pp. 441 (2002). Je nach Person wird eine unterschiedlich starke Extinktion beobachtet.

| | Arm | Achsel | Stirn | Kopfhaut |
|---|---|---|---|---|
| Person A | 0,116 | 0,624 | 0,321 | 0,157 |
| Person B | 0,063 | 0,267 | 0,186 | 0,389 |
| Person C | 0,077 | 0,185 | 0,108 | 0,082 |
| Person D | 0,091 | 0,260 | 0,293 | 0,157 |
| Person E | 0,057 | 0,047 | 0,164 | 0,164 |
| Blindwert (Durchschnitt aus 4 Werten) : 0,049 | | | | |

### Kosmetische Formulierungen:

Es folgen Beispiele für Formulierungen, in denen die erfindungsgemäßen Produkte eingesetzt werden können.

### Formulierung 1:

### Deodorantspray (erfindungsgemäß)

| | |
|---|---|
| Polyglycerin-3 caprylat, enthalten 7 % K caprylat (erfindungsgemäß) | 0,30 % |
| Cyclomethicone | 0,75 % |
| Ethanol | 38,95 % |
| Butan/Propan | 60,00 % |

Die flüssigen Bestandteile werden gemischt und die Formulierung unter Druck in Sprühdosen gefüllt.

### Formulierung 2:

### Deodorantspray (nicht erfindungsgemäß)

| | |
|---|---|
| Triclosan | 0,30 % |
| Silikon | 0,75 % |
| Ethanol | 38,95 % |
| Butan/Propan | 60,00 % |

Die flüssigen Bestandteile werden gemischt und die Formulierung unter Druck in Sprühdosen gefüllt.

### Formulierung 3:

### Clear Deodorant Pumpspray

| Phase A: | |
|---|---|
| Polyglycerin-3 caprylat, | |
| enthalten 7 % K caprylat (erfindungsgemäß) | 0,30 % |
| Trideceth-12 | 2,00 % |
| Dipropylenglykol | 4,00 % |
| Parfüm | 0,90 % |

| Phase B: | |
|---|---|
| Wasser | ad 100,00 |
| Konservierungsmittel | q.s. |
| Zitronensäure (50 %ig) | q.s. |

Die unter Phase A genannten Bestandteile werden unter Rühren in der angegebenen Reihenfolge zusammengefügt und anschließend langsam mit Wasser (Phase B) aufgefüllt. Der pH-Wert wird mit Zitronensäure auf 5,5 eingestellt.

### Formulierung 4:

### O/W-Emulsion (sprühbar)

| Phase A: | |
|---|---|
| Glycerinstearat (und) Ceteth-20 (z.B. TEGINACID® H, Degussa) | 3,00 % |
| Stearylalko Polyglycerin-3 caprylat, | 1,00 % |
| enthalten 7 % K caprylat (erfindungsgemäß) | 0,30 % |
| Dimethicone | 0,50 % |
| Cetearylethylhexanoat | 4,00 % |
| Capryl/Caprin-Triglycerid | 4,00 % |

| Phase B: | |
|---|---|
| Glycerin | 3,00% |
| Wasser | ad 100,00 % |
| Zitronensäure (50 %ig) | pH = 6 bis 7 |
| Konservierungsmittel | q.s. |
| Parfüm | q.s. |

Die Phasen A und B werden auf 70 bis 75 °C erwärmt. Phase A wird unter Rühren zu Phase B gegeben und anschließend homogenisiert. Unter Rühren wird auf 30 °C abgekühlt.

### Wichtig:

Falls Phase A vorgelegt werden sollte, muss Phase B ohne Rühren zugegeben werden.

### Formulierung 5:

### Clear Deodorant Roll On

| Phase A: | |
|---|---|
| Polyglycerin-3 caprylat, | |
| enthalten 7% K caprylat (erfindungsgemäß) | 0,30 % |
| Trideceth-12 | 2,00 % |
| Dipropylenglykol | 2,00 % |
| Parfüm | 0,50 % |
| PEG-14 Dimethicone | 1,00 % |
| Wasser | ad 65,00 % |

| Phase B: | |
|---|---|
| Hydroxyethylcellulose (2 % in Wasser) | 35,00 % |
| Konservierungsmittel | q.s. |
| Zitronensäure (50 %ig) | q.s. |

Die unter Phase A genannten Bestandteile werden unter Rühren in der angegebenen Reihenfolge zusammengefügt. Phase A wird unter Rühren zu Phase B gegeben. Der pH-Wert wird mit Zitronensäure auf 5,5 eingestellt.

### Formulierung 6:

### Clear Deodorant Roll On

| Phase A: | |
|---|---|
| Polyglycerin-3 caprylat, enthalten 7 % K caprylat (erfindungsgemäß) | 0,50 % |
| Laureth-23 | 2,00 % |

| Phase B: | |
|---|---|
| Parfüm | 0,50 % |
| PEG-14 Dimethicone | 0,50 % |
| Alcohol | 20,00 % |
| PEG-7 Gylceryl Cocoate | 1,00 % |
| Wasser | 16,70 % |
| Allatoin | 0,20 % |
| Panthenol | 0,10 % |
| Aluminium Chlorohydrate | 20,00 % |

| Phase C: | |
|---|---|
| Hydroxyethylcellulose | 0,75 % |
| Wasser | 36,75 % |
| Konservierungsmittel | q.s. |

Hydroxyethylcellulose lässt man in Wasser quellen. Die Konservierung wird zugegeben. Die unter Phase A genannten Bestandteile werden auf 50 °C erwärmt. Die unter Phase B genannten Bestandteile werden unter Rühren in die Phase A gegeben. Phase A/B wird dann in Phase C eingerührt.

### Formulierung 7:

### AP/Deo Stick

| Phase A: | |
|---|---|
| Stearyl Alkohol | 23,00 % |
| Hydrogenated Castor Oil | 4,00 % |
| PPG-14 Butyl Ether | 10,00 % |
| Isopropylpalmitat | 16,00 % |
| Laureth-4 | 1,00 % |

| Phase B: | |
|---|---|
| Cyclopentasiloxan | 20,00 % |

| Phase C: | |
|---|---|
| Aluminium Chlorohydrat | 20,00 % |
| Talk | 4,00 % |

| Phase D: | |
|---|---|
| Polyglycerin-3 caprylat, enthalten 7 % K caprylat (erfindungsgemäß) | 1,00 % |
| Parfüm | 1,00 % |

Die unter Phase A genannten Bestandteile werden bei 80 bis 85 °C gerührt bis eine klare Phase erhalten wird. Die unter Phase B genannten Bestandteile werden bei ca. 75 °C eingerührt. Dann werden die unter Phase C und D genannten Bestandteile eingerührt.

### Formulierung 8:

### Clear Deodorant Pumpspray

| Phase A: | |
|---|---|
| Polyglycerin-3 caprylat, | |
| enthalten 7 % K caprylat (erfindungsgemäß) | 0,50 % |
| Laureth-23 | 3,00 % |

| Phase B: | |
|---|---|
| Parfüm | 0,50 % |
| Bis-PEG/PPG-20/20 Dimethicone | 0,50 % |
| Wasser | 94,00 % |
| Allatoin | 0,20 % |
| Panthenol | 0,10 % |
| PEG-7 Gylceryl Cocoate | 1,00 % |
| Tri-Natrium Citrate Dihydrat | 0,20 % |
| Konservierungsmittel | q.s. |
| Zitronensäure | q.s. |

Die unter Phase A genannten Bestandteile werden auf 50 °C erwärmt. Die unter Phase B genannten Bestandteile werden unter Rühren in der angegebenen Reihenfolge in Phase A gegeben. Der pH-Wert wird mit Zitronensäure auf 5,5 eingestellt.

### Formulierung 9:

### Anionischer Haushaltsreiniger (Konzentrat)

| Phase A: | |
|---|---|
| Polyglycerin-3 caprylat, enthalten 7 % K caprylat (erfindungsgemäß) | 4,00 % |
| Ethanol | 10,00 % |
| Trideceth-12 | 5,00 % |
| Cocamidopropylbetain (- 38 % Wirkstoffanteil) | 13,20 % |
| Natriumlaurylethersulfat | 35,80 % |

| Phase B: | |
|---|---|
| Wasser | ad 100,00 % |

Die unter Phase A genannten Bestandteile werden unter Rühren in der angegebenen Reihenfolge zusammengefügt und anschließend langsam mit Wasser (Phase B) aufgefüllt.

### Formulierung 10:

### Flüssigseife

| | |
|---|---|
| Sodium Laureth Sulfate | 25,0 % |
| Polyglyceryl-3 Caprylate enthalten 7 % K caprylat (erfindungsgemäß) | 0,5 % |
| PEG-7 Glyceryl Cocoate | 1,5 % |
| Parfum | 0,5 % |
| Wasser | 62,5 % |
| Cocamidopropyl Betaine | 8,0 % |
| PEG-18 Glyceryl Oleate/Cocoate | 2,0 % |
| Natrium Chloride | q.s. |
| Konservierungsmittel | q.s. |

Alle Komponenten werden in der angegebenen Reihenfolge gemischt.

### Formulierung 11:

### Zahnpasta

| | |
|---|---|
| Wasser | 38,25 % |
| Natrium Benzoate | 0,2 % |
| Hydroxyethyl Cellulose | 1,8 % |
| Xylit | 0,3 % |
| Sorbitol (70 %) | 12,0 % |
| Cocamidopropyl Betaine | 2,45 % |
| Dimethicone Copoyol | 2,0 % |
| Polyglyceryl-3 caprylate enthalten 7 % K caprylat (erfindungsgemäß) | 0,5 % |
| Natrium Fluoride | 0,2 % |
| Calcium Phosphate | 33,0 % |
| Silica | 8,0 % |
| Titandioxid mikro | 0,2 % |
| PEG-30 Glyceryl Stearate | 0,5 % |
| Aromaoil | 0,6 % |

Das Natriumbenzoat wird in Wasser gelöst, die Hydroxyethyl Cellulose zugegeben. Nachdem diese hinreichend gequollen ist werden die anderen Komponenten in gegebener Reihenfolge eingearbeitet.

### Formulierung 12:

### Anti-Schuppenschampoo

| | |
|---|---|
| Natrium Laureth Sulfate, 28 % | 30 % |
| Disodium Cocoamphodiacetate | 8,0 % |
| Undecylenamidopropyl Betain | 4,0 % |
| Polyglyceryl-3 caprylate enthalten 7 % K caprylat (erfindungsgemäß) | 0,5 % |
| Wasser | 57,08 % |
| Zitronensäure Monohydrate | 0,42 % |
| Konservierungsmittel, Parfum | q.s. |

Die Komponenten werden in der gegebenen Reihenfolge gemischt. Der pH-Wert wird mit Zitronensäure auf ca. 6 eingestellt.

### Kosmetischer Anwendungstest:

Zur Anwendung kommen zwei Formulierungen. Diese sind Formulierung 1 und 2. Der Achselgeruch von 20 Probanden wird vor und nach der Anwendung der Formulierung durch drei Experten geprüft. Im Einzelnen beinhaltet der Test folgende Schritte:
1. Die Achselhöhle wird mit Seife gewaschen, der Geruch wird durch Experten bewertet,
2. Das Produkt wird einmal in einer Achselhöhle angewendet. Nach 6 und 24 h wird der Geruch geprüft und der Unterschied bewertet.

Das Ergebnis dieser Untersuchung ist, dass sowohl nach 6 als auch nach 24 stündiger Anwendung eine deutliche Verbesserung des Geruches der erfindungsgemäß behandelten Achselhöhle (Formulierung 1) festgestellt wird. Eine Verbesserung ist ebenso gegenüber der unbehandelten Achsel festzustellen. Die mit der Formulierung nach dem Stand der Technik behandelte Achsel (Formulierung 2) zeigt keine Verbesserung des Geruchs, lediglich eine Verbesserung gegenüber der unbehandelten Achsel.

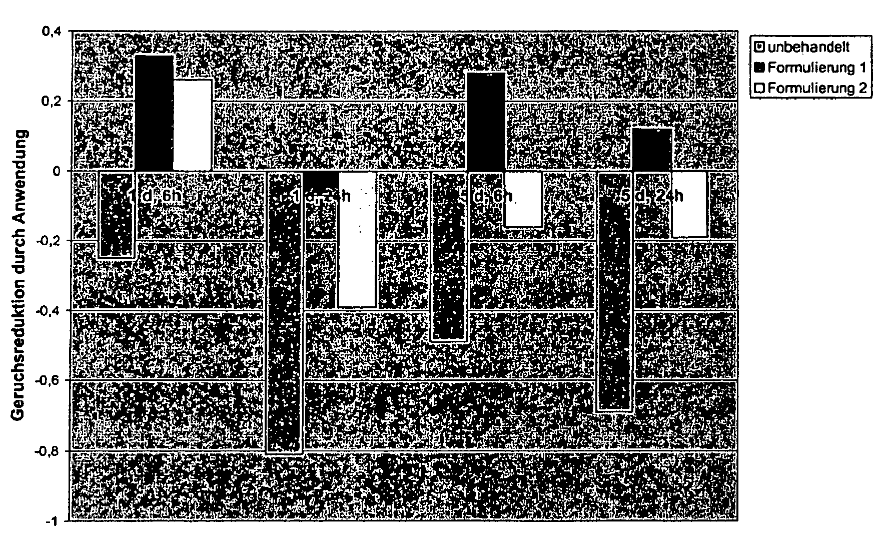

### Konservierung eines Lebensmittels: (nicht erfindungsgemäß)

Kartoffelsalat bestehend aus 750 g gekochten und klein geschnittenen Kartoffeln, 25 g klein geschnittener Zwiebeln, 1,2 g Kochsalz, 10 ml Essig (enthaltend 6 % Essigsäure) und 200 g Mayonaise wird mit 0,5 % des Polyglycerinesters aus Beispiel 4 versetzt. Der Kartoffelsalat wurde zur Überprüfung auf Bakterien und Hefen 72 Stunden bei 30 °C gelagert. Danach wurden die folgenden Keimzahlen bestimmt:

| | |
|---|---|
| Kartoffelsalat ohne Polyglycerinester: | 1,2 x 10⁶ Keine/ml |
| Kartoffelsalat mit Polyglycerinester: | 1,3 x 10³ Keine/ml |

Zur Überprüfung auf Hefen und Pilze wurde der Kartoffelsalat 72 Stunden bei 25 °C gelagert. Danach wurden die folgenden Keimzahlen bestimmt:

| | |
|---|---|
| Kartoffelsalat ohne Polyglycerinester: | 6,7 x 10⁴ Keine/ml |
| Kartoffelsalat mit Polyglycerinester: | 2,5 x 10¹ Keine/ml |

Der Kartoffelsalat ohne Polyglycerinester zeigte nach 96 Stunden Lagerung deutlich sichtbaren bläulichen Schimmel, während der Kartoffelsalat mit Polyglycerinester optisch unverändert war.

## Patentansprüche

1. Mittel zur Bekämpfung von Mikroorganismen, **dadurch gekennzeichnet, dass** sie einen wirksamen Gehalt an Mischungen von Fettsäureestern des Polyglycerins und von Salzen kurzkettiger Monocarbonsäuren ausgewählt aus Caprylsäure enthalten.

2. Mittel gemäß Anspruch 1, **dadurch gekennzeichnet, dass** sie einen wirksamen Anteil an Mono- und Diestern des Mono-, Di- und Triglycerins enthalten.

3. Mittel gemäß Anspruch 1, **dadurch gekennzeichnet, dass** das Verhältnis von Fettsäureestern zu Monocarbonsäuresalzen im Bereich von 80 : 20 bis 99 :-1 liegt.

4. Mittel gemäß Anspruch 1, **dadurch gekennzeichnet, dass** als Säuren und Säurederivate für die Veresterung geradkettige oder verzweigte, gegebenenfalls OH-Gruppen und/oder Doppelbindungen enthaltende Fettsäuren mit 6 bis 14 C-Atomen in der Hauptkette verwendet werden.

5. Verwendung von Mitteln gemäß Anspruch 1 zur nicht-therapeutischen Bekämpfung von grampositiven Bakterien, gramnegativen Bakterien, Mykobakterien, Dermatophyten, Spross- und Fadenpilzen, Viren und Sporen.

6. Verwendung von Mitteln gemäß Anspruch 1 zur Herstellung von Desinfektionsmitteln, Desinfektionsreinigern, Sterilisationsmitteln, Antiseptika und Konservierungsmitteln.

7. Verwendung von Mitteln gemäß Anspruch 1 zur Konservierung von Lebensmitteln.

8. Verwendung von Mitteln gemäß Anspruch 1 zur antimikrobiellen Ausrüstung von Lebensmittelverpackungen zur Verbesserung der Haltbarkeit des Inhalts.

9. Verwendung von Mitteln gemäß Anspruch 1 zur Herstellung von kosmetischen Formulierungen für den Bereich der Körperreinigung und Körperpflege.

10. Verwendung von Mitteln gemäß Anspruch 1 zur Herstellung Kosmetischer Formulierungen gegen Körpergeruch und gegen Schuppenbildung.

11. Verwendung von Mitteln gemäß Anspruch 1 zur Herstellung von kosmetischen Formulierungen gegen unreine Haut und leichte Formen der Akne.

12. Verwendung von Mitteln gemäß Anspruch 1 zur Herstellung von Zahnpflegeprodukten, wie Zahncreme und Mundwasser.

## Claims

1. Compositions for controlling microorganisms, **characterized in that** they comprise an effective content of mixtures of fatty acid esters of polyglycerol and of salts of short-chain monocarboxylic acids selected from caprylic acid.

2. Compositions according to Claim 1, **characterized in that** they comprise an effective content of monoesters and diesters of mono-, di- and/or triglycerol.

3. Compositions according to Claim 1, **characterized in that** the ratio of fatty acid esters to monocarboxylic acid salts is in the range from 80:20 to 99:1.

4. Compositions according to Claim 1, **characterized in that** the acids and acid derivatives used for the esterification are straight-chain or branched fatty acids having 6 to 14 carbon atoms in the main chain and optionally containing OH groups and/or double bonds.

5. Use of compositions according to Claim 1 for non-therapeutic control of Gram-positive bacteria, Gram-negative bacteria, mycobacteria, dermatophytes, yeast and hyphal fungi, viruses and spores.

6. Use of compositions according to Claim 1 for producing disinfectants, disinfectant cleaners, sterilizing compositions, antiseptics and preservatives.

7. Use of compositions according to Claim 1 for preserving foods.

8. Use of compositions according to Claim 1 for the antimicrobial finishing of food packagings to improve the shelf -life of the contents.

9. Use of compositions according to Claim 1 for producing cosmetic formulations for the field of body cleansing and body care.

10. Use of compositions according to Claim 1 for producing cosmetic formulations against body odor and against formation of dandruff.

11. Use of compositions according to Claim 1 for producing cosmetic formulations against blemished skin and mild forms of acne.

12. Use of compositions according to Claim 1 for producing dental care products, such as toothpaste and mouthwash.

## Revendications

1. Agents pour la lutte contre des micro-organismes, **caractérisés en ce qu'**ils ont une teneur efficace en mélanges d'esters d'acides gras du polyglycérol et de sels d'acides monocarboxyliques à courte chaîne, choisis parmi ceux de l'acide caprylique.

2. Agents selon la revendication 1, **caractérisé en ce qu'**ils ont une teneur efficace en mono- et diesters du mono-, di- et triglycérol.

3. Agents selon la revendication 1, **caractérisé en ce que** le rapport des esters d'acides gras aux sels d'acides monocarboxyliques se situe dans la plage allant de 80 : 20 à 99 : 1.

4. Agents selon la revendication 1, **caractérisé en ce que** pour l'estérification on utilise comme acides et dérivés d'acides des acides gras à chaîne droite ou ramifiée ayant de 6 à 14 atomes de carbone dans la chaîne principale, contenant éventuellement des groupes OH et/ou des doubles liaisons.

5. Utilisation d'agents selon la revendication 1, pour la lutte non thérapeutique contre des bactéries à Gram positif, des bactéries à Gram négatif, des mycobactéries, des dermatophytes, des levures et des champignons filamenteux, des virus et des spores.

6. Utilisation d'agents selon la revendication 1, pour la fabrication de désinfectants, de produits de nettoyage désinfectants, de produits pour stérilisation, d'antiseptiques et de conservateurs.

7. Utilisation d'agents selon la revendication 1, pour la conservation de produits alimentaires.

8. Utilisation d'agents selon la revendication 1, pour le traitement antimicrobien d'emballages de produits alimentaires pour l'amélioration de l'aptitude au stockage du contenu.

9. Utilisation d'agents selon la revendication 1, pour la fabrication de compositions cosmétiques destinées au secteur du nettoyage corporel et du soin du corps.

10. Utilisation d'agents selon la revendication 1, pour la fabrication de compositions cosmétiques contre l'odeur corporelle et contre la formation de pellicules.

11. Utilisation d'agents selon la revendication 1, pour la fabrication de compositions cosmétiques contre les impuretés de la peau et les formes légères de l'acné.

12. Utilisation d'agents selon la revendication 1, pour la fabrication de produits pour le soin des dents, tels que des crèmes dentifrices et des bains de bouche.
